(19) Europäisches Patentamt

European Patent Office

Office européen des brevets

(11) **EP 1 655 377 A1**

(12) **EUROPÄISCHE PATENTANMELDUNG**

(43) Veröffentlichungstag:
**10.05.2006 Patentblatt 2006/19**

(51) Int Cl.:
*C12Q 1/68* *(2006.01)*    *G06F 19/00* *(2006.01)*

(21) Anmeldenummer: 04090133.2

(22) Anmeldetag: **06.04.2004**

(84) Benannte Vertragsstaaten:
**AT BE BG CH CY CZ DE DK EE ES FI FR GB GR HU IE IT LI LU MC NL PL PT RO SE SI SK TR**
Benannte Erstreckungsstaaten:
**AL HR LT LV MK**

(71) Anmelder: **Epigenomics AG**
**10178 Berlin (DE)**

(72) Erfinder:
• **Kluth, Antje**
**22049 Hamburg (DE)**
• **Schuster, Matthias**
**13158 Berlin (DE)**

• **Schmitt, Armin**
**12203 Berlin (DE)**
• **Habighorst, Dirk**
**13187 Berlin (DE)**
• **Gütig, David**
**13357 Berlin (DE)**

(74) Vertreter: **Schubert, Klemens**
**Müller & Schubert**
**Patentanwälte**
**Neue Promenade 5**
**10178 Berlin (DE)**

(54) **Verfahren zur Quantifizierung methylierter DNA**

(57) Das erfindungsgemäße Verfahren betrifft ein Verfahren zur Quantifizierung von methylierter DNA. Hierzu wird die zu untersuchende DNA zunächst so umgewandelt, dass Cytosin in Uracil umgewandelt wird, während 5'Methylcytosin unverändert bleibt. Anschließend wird die umgewandelte DNA mittels einer Real-Time-PCR-PCR amplifiziert. Dabei wird jeweils eine Sonde eingesetzt, die für den methylierten bzw. den unmethylierten Zustand der DNA spezifisch ist. Aus dem Verhältnis der Signalintensitäten der Sonden oder aus den Ct-Werten läßt sich der Methylierungsgrad der untersuchten DNA berechnen. Das erfindungsgemäße Verfahren eignet sich insbesondere zur Diagnose und Prognose von Krebs und anderen mit einer Veränderung des Methylierungsstatus assoziierten Krankheiten sowie zur Vorhersage von Arzneimittelwirkungen.

EP 1 655 377 A1

**Beschreibung**

Die vorliegende Erfindung betrifft ein Verfahren zur Quantifizierung von methylierten Cytosinpositionen in DNA. 5-Methylcytosin ist die häufigste kovalent modifizierte Base in der DNA eukaryotischer Zellen. Sie spielt eine wichtige biologische Rolle, u.a. bei der Transkriptionsregulation, beim genetischen Imprinting und in der Tumorgenese (zur Übersicht: Millar et al.: Five not four: History and significance of the fifth base. In: The Epigenome, S. Beck and A. Olek (eds.), Wiley-VCH Verlag Weinheim 2003, S. 3-20). Die Identifizierung von 5-Methylcytosin ist insbesondere für die Krebsdiagnostik von erheblichen Interesse. Ein Nachweis von Methylcytosin ist allerdings schwierig, da Cytosin und Methylcytosin das gleiche Basenpaarungsverhalten aufweisen. Die herkömmlichen, auf Hybridisierung beruhenden DNA-Analyseverfahren sind daher nicht anwendbar. Dementsprechend arbeiten die gängigen Methoden zur Methylierungsanalyse nach zwei unterschiedlichen Prinzipien. Zum einen werden methylierungsspezifische Restriktionsenzyme benutzt, zum anderen erfolgt eine selektive chemische Umwandlung von nicht-methylierten Cytosinen in Uracil (sog.: Bisulfit-Behandlung, siehe etwa: DE 101 54 317 A1; DE 100 29 915 A1). Die enzymatisch oder chemisch vorbehandelte DNA wird dann meist amplifiziert und kann auf unterschiedliche Weise analysiert werden (zur Übersicht: WO 02/072880 S. 1 ff; Fraga and Estella: DNA methylation: a profile of methods and applications. Biotechniques. 2002 Sep;33(3):632, 634, 636-49.). Zur sensitiven Analyse wird die chemisch vorbehandelte DNA üblicherweise mittels eines PCR-Verfahrens amplifiziert. Eine selektive Amplifikation nur der methylierten (bzw. bei umgekehrten Ansatz: unmethylierten) DNA kann über die Verwendung methylierungsspezifischer Primer oder Blocker gewährleistet werden (sog. methylierungssensitive PCR/MSP bzw. "Heavy Methyl-Verfahren , vgl.: Herman et al.: Methylation-specific PCR: a novel PCR assay for methylation status of CpG islands. Proc Natl Acad Sci U S A. 1996 Sep 3;93 (18):9821-6 Cottrell et al.: A real-time PCR assay for DNA-methylation using methylation-specific blockers. Nucl. Acids. Res. 2004 32: e10). Auf der anderen Seite ist es auch möglich, die DNA zunächst methylierungsunspezifisch zu amplifizieren und dann die Amplifikate mittels methylierungsspezifischer Sonden zu analysieren (zur Übersicht: Trinh et al.: DNA methylation analysis by MethyLight technology. Methods. 2001 Dec;25(4): 456-62). Die genannten PCR-Verfahren sind auch als Real-Time-PCR-Varianten anwendbar. Diese ermöglichen es, den Methylierungsstatus direkt im Verlauf der PCR nachzuweisen, ohne dass eine nachfolgende Analyse der Produkte erforderlich wäre ("MethyLight" - WO00/70090; US 6,331,393; Trinh et al. 2001, a.a.o.).

Eine Quantifizierung des Methylierungsgrades ist für verschiedene Anwendungen erforderlich, etwa für Klassifizierungen von Tumoren, für prognostische Aussagen oder für die Vorhersage von Arzneimittelwirkungen. Es sind unterschiedliche Verfahren zur Quantifizierung des Methylierungsgrades bekannt. Zum Teil erfolgt dabei zunächst eine Amplifikation der DNA, etwa bei Ms-SNuPE, bei Hybridisierungen auf Microarrays, bei Hybridisierungsassays in Lösung oder bei der direkten Bisulfit-Sequenzierung (zur Übersicht: Fraga and Estella 2002, a.a.o.). Ein Problem bei diesen "Endpunktanalysen" besteht darin, dass die Amplifikation u.a. aufgrund Produkthemmung, Enzyminstabilität und Konzentrationsabnahme der Reaktionskomponenten ungleichmäßig erfolgen kann. Eine Korrelation zwischen der Menge an Amplifikat und der Menge an eingesetzter DNA ist daher nicht immer gegeben. Die Quantifizierung wird daher fehleranfällig (vgl.: Kains: The PCR plateau phase - towards an understanding of its limitations. Biochem. Biophys. Acta 1494 (2000) 23-27). Die auf einer Real-Time-PCR basierende Schwellenwertanalyse bestimmt die Menge an Amplifikat dagegen nicht am Ende der Amplifikation, sondern in der exponentiellen Amplifikationsphase. Diese Methode setzt voraus, dass die Amplifikationseffizienz in der exponentiellen Phase konstant ist. Der sog. Schwellenwert Ct ist ein Maß für denjenigen PCR-Zyklus, bei dem das Signal in der exponentiellen Phase der Amplifikation zum ersten Mal größer als das Hintergrundrauschen ist. Die absolute Quantifizierung erfolgt dann über einen Vergleich des Ct-Werts der untersuchten DNA mit dem Ct-Wert eines Standards (vgl.: Trinh et al. 2001, a.a.o.; Lehmann et al.: Quantitative assessment of promoter hypermethylation during breast cancer development. Am J Pathol. 2002 Feb;160(2):605-12). Ein Problem der Ct-Analyse besteht darin, dass bei hohen DNA-Konzentrationen nur eine geringe Auflösung erreicht werden kann. Das gleich gilt, wenn hohe Methylierungsgrade über PMR-Werte ermittelt werden sollen (vgl. zu PMR-Werten: Eads et al., CANCER RESEARCH 61, 3410-3418, April 15, 2001.) Zudem ist für diese Art der Ct-Analyse auch die Amplifkation eines Referenzgens, etwa des ß-Aktin-Gens, erforderlich (vgl.: Trinh et al 2001, a.a.o.).

Im folgenden wird ein neues Real-Time-PCR-Verfahren zur quantitativen Methylierungsanalyse beschrieben. Dabei erfolgt eine methylierungsunspezifische, konvertierungsspezifische Amplifikation der Ziel-DNA. Die Detektion der Amplifikate erfolgt über die Hybridisierung zweier unterschiedlicher methylierungsspezifischer Real-Time-PCR-Sonden. Dabei ist die eine Sonde spezifisch für den methylierten Zustand, während die andere Sonde spezifisch für den unmethylierten Zustand ist. Die beiden Sonden tragen unterschiedliche Fluoreszenzfarbstoffe. Innerhalb bestimmter PCR-Zyklen kann über das Verhältnis der Signalintensitäten der beiden Sonden eine Quantifizierung des Methylierungsgrades erfolgen. Alternativ können auch die Ct's beider Fluoreszenskanäle zur Quantifizierung der Methylierung herangezogen werden. In beiden Fällen ist eine Quantifizierung des Methylierungsgrades möglich, ohne dass die absolute DNA-Menge bestimmt werden muß. Eine gleichzeitige Amplifikation eines Referenzgens oder eine Bestimmung der PMR-Werte ist daher nicht erforderlich. Zudem liefert das erfindungsgemäße Verfahren sowohl für hohe wie auch für geringe DNA-Mengen sowie für hohe und geringe Methylierungsgrade verläßliche Werte.

Aufgrund der besonderen Bedeutung der Cytosinmethylierung besteht ein großes technisches Bedürfnis an quantitativen Verfahren zur Methylierungsanalyse. Das erfindungsgemäße Verfahren überwindet die oben beschriebenen Nachteile des Standes der Technik und stellt daher einen wichtigen technischen Fortschritt dar.

**Beschreibung**

Das erfindungsgemäße Verfahren zur Quantifizierung von methylierter DNA ist dadurch gekennzeichnet, dass folgende Schritte durchgeführt werden:

  a) die zu untersuchende DNA wird so umgesetzt, dass 5- Methylcytosin unverändert bleibt, während unmethy- liertes Cytosin in Uracil oder in eine andere Base umgewandelt wird, die sich im Basenpaarungsverhal- ten von Cytosin unterscheidet,
  b) die umgewandelte DNA wird in Gegenwart zweier Real- Time-Sonden amplifiziert, wobei die eine Sonde spe- zifisch für den methylierten Zustand und die andere Sonde spezifisch für den unmethylierten Zustand der DNA ist,
  c) zu unterschiedlichen Zeitpunkten wird festgestellt, inwieweit eine Hybridisierung der Sonden an die Amplifikate erfolgt ist,
  d) der Methylierungsgrad der untersuchten DNA wird be- stimmt.

Im ersten Schritt dieser Ausführungsform wird die zu untersuchende DNA mit einer Chemikalie oder mit einem Enzym so umgesetzt, dass 5-Methylcytosin unverändert bleibt, während unmethyliertes Cytosin in Uracil oder in eine andere Base umgewandelt wird, die sich im Basenpaarungsverhalten von Cytosin unterscheidet. Dabei kann die zu untersuchende DNA je nach diagnostischer oder wissenschaftlicher Fragestellung aus unterschiedlichen Quellen stammen. Für diagnostische Fragestellungen dienen als Ausgangsmaterial bevorzugt Gewebeproben, aber auch Körperflüssigkeiten, insbesondere Serum. Möglich ist auch, die DNA aus Sputum, Stuhl, Urin oder Gehirn-Rückenmarks-Flüssigkeit zu verwenden. Vorzugsweise wird die DNA zunächst aus der biologischen Probe isoliert. Die DNA-Extraktion erfolgt nach Standardmethoden, aus Blut etwa unter Verwendung des Qiagen UltraSens DNA Extraktions-Kits. Die isolierte DNA kann dann z.B. durch Umsatz mit Restriktionsenzymen fragmentiert werden. Die Reaktionsbedingungen und die in Frage kommenden Enzyme sind dem Fachmann bekannt und ergeben sich etwa aus den von den Herstellern mitgelieferten Protokollen. Anschließend wird die DNA chemisch oder enzymatisch umgewandelt.

Bevorzugt erfolgt einen chemische Umsetzung mittels Bisulfit. Die Bisulfitumwandlung ist dem Fachmann in unterschiedlichen Variationen bekannt (siehe etwa: Frommer et al.: A genomic sequencing protocol that yields a positive display of 5-methylcytosine residues in individual DNA strands. Proc Natl Acad Sci U S A. 1992 Mar 1;89 (5):1827-31; Olek, A modified and improved method for bisulphite based cytosine methylation analysis. Nucleic Acids Res. 1996 Dec 15; 24 (24) :5064-6. ; DE 100 29 915; DE 100 29 915) . Besonders bevorzugt erfolgt die Bisulfitumwandlung in Gegenwart von denaturierenden Lösemitteln, etwa Dioxan, und eines Radikalfängers (vgl.: DE 100 29 915). In einer anderen bevorzugten Ausführungsform wird die DNA nicht chemisch, sondern enzymatisch umgewandelt. Dies ist etwa durch Einsatz von Cytidin-Deaminasen denkbar, die unmethylierte Cyidine schneller umsetzen als methylierte Cytidine. Ein entsprechendes Enzym ist kürzlich identifiziert worden (Bransteitter et al.: Activation-induced cytidine deaminase deaminates deoxycytidine on singlestranded DNA but requires the action of RNase. Proc Natl Acad Sci U S A. 2003 Apr 1;100(7):4102-7).

Im zweiten Schritt des erfindungsgemäßen Verfahrens wird die umgewandelte DNA in Gegenwart zweier Real-Time-Sonden amplifiziert, wobei die eine Sonde spezifisch für den methylierten Zustand und die anderer Sonde spezifisch für den nicht-methylierten Zustand ist. Bevorzugt erfolgt dabei eine Amplifikation mittels eines exponentiellen Amplifikationsverfahrens, besonders bevorzugt mittels einer PCR. Für die Amplifikation werden Primer verwendet, die spezifisch für die chemisch oder enzymatisch umgewandelte DNA sind. Bevorzugt werden dabei methylierungsunspezifische Primer eingesetzt, d.h. Primer, die über keine CG- bzw. kein methylierungsspezifisches TG- oder CA-Dinukleotid verfügen. Mit diesen Primern erfolgt eine gleichmäßige Amplifikation methylierter und unmethylierter DNA. Es ist jedoch auch möglich, einen größeren Sequenzbereich methylierungsspezifisch zu amplifizieren und dabei bestimmte Cytosinpositionen innerhalb dieser Sequenz mit Hilfe des erfindungsgemäßen Verfahrens zu quantifizieren. Das Design von methylierungsspezifischen und methylierungsunspezifischen Primern und die PCR-Reaktionsbedingungen gehören zum Stand der Technik (vgl.: etwa: US Patent 6,331,393; Trinh et al 2001, a.a.o.).

Die Amplifikation erfolgt in Gegenwart zweier unterschiedlicher Sonden, wobei die eine Sonde spezifisch für den methylierten Zustand der DNA ist, während die andere Sonde spezifisch für den unmethylierten Zustand der DNA ist. Die methylierungsspezifischen Sonden tragen dementsprechend mindestens ein CpG-Dinukleotid, während die nicht-methylierungsspezifischen Sonden über mindestens ein spezifisches TG- bzw. CA-Dinukleotid verfügen. Bevorzugt tragen die Sonden drei spezifische Dinukleotide. Bei den Sonden handelt es sich um Real-Time-Sonden. Hierunter werden im folgenden Sonden verstanden, die es erlauben, die Amplifikate bereits während der Amplifikation nachzuweisen. Dem Fachmann sind unterschiedliche Real-Time-PCR-Varianten verwandt, etwa Lightcycler-, Taqman-, Sunrise-, Molecular Beacon- oder Eclipse-Sonden. Einzelheiten zum Aufbau und zum Nachweis dieser Sonden gehören zum Stand der Technik (vgl.: US Patent 6,331,393 mit weiteren Nachweisen). So kann das Design der Sonden etwa über die "PrimerExpress"-Software von Applied Biosystems (für Taqman-Sonden) oder über MGB Eclipse Design Software von E-poch Biosciences (für Eclipse-Sonden) erfolgen. Bevorzugt werden Taqman-Sonden verwendet, die besonders bevozugt in Kombination mit Minor Groove Bindern (MGB) eingesetzt werden.

Die Amplifikation erfolgt bevorzugt zusammen mit beiden Sonden in einem Gefäß, so dass die Reaktionsbedingungen für beide Sonden identisch sind. Diese Ausführungsform führt zudem zu einer erhöhten Spezifität, da die Sonden um die Bindungsstellen kompetitieren. Es ist allerdings erforderlich, dass die beiden Sonden unterschiedliche Markierungen tragen. Es ist auf der anderen Seite auch möglich, die Amplifkationen in unterschiedlichen Gefäßen durchzuführen. Hierdurch lassen sich störende Wechselwirkungen zwischen den Fluoreszenzfarbstoffen vermeiden.

Im dritten Schritt des erfindungsgemäßen Verfahrens wird zu unterschiedlichen Zeitpunkten festgestellt, inwieweit eine Hybridisierung der Sonden an die Amplifikate erfolgt ist. Der Nachweis der Hybridisierungen erfolgt während der einzelnen Amplifikationszyklen. Die Detektion erfolgt dabei in Abhängigkeit von den eingesetzten Sonden nach dem Stand der Technik.

Im vierten Schritt des erfindungsgemäßen Verfahrens wird der Methylierungsgrad der untersuchten DNA bestimmt. Dies kann über unterschiedliche Ausführungsformen geschehen. In einer bevorzugten Ausführungsform wird aus dem Verhältnis der Signalintensitäten der beiden Sonden der Methylierungsgrad der untersuchten DNA bestimmt. Dies kann etwa über die folgende Formel erfolgen:

$$M = 100 * I_{CG} / (I_{CG} + I_{TG})$$

Dabei handelt es sich bei $I_{CG}$ um die Signalintensität der für den methylierten Zustand spezifischen Sonde und bei bei $I_{TG}$ um die Signalintensität der für den nicht-methylierten Zustand spezifischen Sonde.

Besonders bevorzugt werden die Signalintensitäten während eines PCR-Zyklusses in der exponentiellen Amplifikationsphase der PCR miteinander in Verhältnis gesetzt. Bevorzugt erfolgt eine Berechnung in der Nähe des Zyklusses, bei dem die Amplifikation ihre maximale Steigung erreicht. Dies entspricht dem Wendepunkt der Fluoreszenzintensitätskurven bzw. des Maximums ihrer ersten Ableitung. Die Berechnung erfolgt dabei zu einem Zeitpunkt, der bevorzugt bis zu fünf Zyklen vor oder nach dem Wendepunkt, besonders bevorzugt bis zu zwei Zyklen vor oder nach dem Wendepunkt und ganz besonders bevorzugt bis zu einem Zyklus vor oder nach dem Wendepunkt liegt. In der besten Ausführungsform findet die Berechnung direkt im Wendepunkt statt.

Für den Fall, dass die Wendepunkte der beiden Kurven in unterschiedlichen Zyklen liegen, erfolgt die Berechnung bevorzugt bei dem Wendepunkt der Kurve, die zu diesem Zeitpunkt das höchste Signal aufweist.

Die Bestimmung der Wendepunkte erfolgt bevorzugt über die erste Ableitung der Fluoreszenzintensitätskurven. Vorzugsweise werden die Ableitungen zunächst einer Glättung unterzogen ("Spline"). Einzelheiten sind dem Fachmann bekannt.

In einer anderen bevorzugten Ausführungsform erfolgt die Berechnung des Methylierungsgrades nicht über das Verhältnis der Fluoreszenzintensitäten, sondern über das Verhältnis von Schwellenwerten, bei denen eine gewisse Signalintensität überschritten wird, etwa bei Ct-Werten (s.o.). Die Bestimmung von Ct-Werten gehört zum Stand der Technik (vgl.: Trinh et al, a.a.o., 2002). Der Methylierungsgrad läßt sich dann über die folgende Formel bestimmen: Methylierungsgrad = 100/ $(1+2^{\Delta Ct})$.

Daneben ist es denkbar, andere Kriterien zur Berechnung des Methylierungsgrades zu verwenden, etwa die Fläche unter den Fluoreszenzkurven (area under the curve) oder die maximale Steigung der Kurven.

Eine Quantifizierung über die oben beschriebene Verfahren ist besonders gut möglich, wenn die Assaybedingungen diesbezüglich zuvor optimiert wurden. Eine Optimierung erfolgt mit unterschiedlichen Methylierungsstandards (etwa mit 0%, 5%, 10%, 25%, 50%, 75% und 100% Methylierungsgrad). Als Standard wird bevorzugt DNA verwendet, die die gesamte genomische DNA oder einen repräsentativen Teil hiervon abdeckt. Die unterschiedlichen Methylierungsgrade erhält man durch entsprechende Mischungen aus methylierter und nicht-methylierter DNA. Die Herstellung methylierter DNA ist relativ einfach über die Verwendung der SssI-Methylase möglich. Dieses Enzym überführt im Sequenzkontext CG alle nicht-methylierten Cytosine in 5-Methylcytosin. Als vollständig nicht-methylierte DNA kann Sperma-DNA verwendet werden, die über einen nur geringen Methylierungsgrad verfügt (vgl.: Trinh et al. 2001, a.a.o.). Bevorzugt erfolgt aber die Herstellung nicht-methylierter DNA mittels einer sog. genomweiten Amplifikation (WGA - whole genome amplification, zur Übersicht: Hawkins et al.: Whole genome amplification--applications and advances. Curr Opin Biotechnol. 2002 Feb; 13 (1): 65-7).WGA). Hierbei werden weite Teile des Genoms mittels "Random" oder degenerierter Primer amplifiziert. Da in der Amplifikation nur unmethylierte Cytosinnukleotide angeboten werden, resultiert nach mehreren Amplifikationszyklen eine vollständig unmethylierte DNA. Bevorzugt erfolgt dabei eine "Multiple Displacement Amplification" mittels der φ29 Polymerase (MDA, vgl.: Dean et al. 2002 a.a.o.; US Patent 6,124,120). Entsprechend hergestellte DNA ist über unterschiedliche komerzielle Anbieter verfügbar ("GenomiPhi" von Amersham Biosciences, www4.amershambiosciences.com; "Repli-g" von Molecular Staging, www.molecularstaging.com). Die Herstellung von Methylierungsstandards ist sehr detailliert in der europäischen Patentanmeldung 04 090 037.5 beschrieben (Anmeldedatum: 5. Feb. 2004; Anmelder: Epigenomics AG). Indem der Quotient der Signale, die für den methylierten Zustand detektiert werden, und der Summe der Signale, die für den methylierten und den unmethylierten Zustand detektiert werden, gebildet wird, erhält man die gemessene Methylierungsrate. Trägt man diese gegen die theoretischen Methylierungraten (entsprechend dem Anteil methylierter DNA in den definierten Mischungen) auf und ermittelt die Regression, die durch die Messpunkte geht, erhält man eine Kalibrierungskurve. Bevorzugt erfolgt eine Kalibrierung mit unterschiedlichen Mengen an DNA, etwa mit 0,1; 1 und 10 ng DNA pro Ansatz.

Assays eignen sich besonders für die Quantifizierung über das erfindungsgemäße Verfahren, wenn die Kalibrierungskurven für den Zeitpunkt der exponentiellen Amplifkation möglichst über einen y-Achsenabschnitt bei Null verfügen. Benachbarte Methylierungszustände sollten mit einem hohen Fischer-Score (bevorzugt über 1, besonders bevorzugt über 3) unterschieden werden.. Vorteilhaft ist weiterhin, wenn die über einen möglichst geringen y-Achsenabschnitt und einen möglichst hohen Fischer-Score (bevorzugt über 1, besonders bevorzugt über 3) verfügen. Vorteilhaft ist weiterhin, wenn die Kurven eine Steigung und eine Regression nahe dem Wert 1 haben.

Die Assays können diesbezüglich mittels Variation der Primer, der Sonden, des Temperaturprogramms und der weiteren Reaktionsparameter über Standardversuche optimiert werden.

Wie bereits oben erwähnt, ist die Bestimmung der Methylierungsrate mit dem erfindungsgemäßen Verfahren unabhängig von einer Standardkurve möglich. Wird jedoch eine Standardkurve angefertigt, so läßt sich mittels des erfindungsgemäßen Verfahrens auch sehr einfach der absolute Gehalt an methylierter DNA bestimmen.

Eine besonders bevorzugte Verwendung des erfindungsgemäßen Verfahrens liegt in der Diagnose oder Prognose von Krebserkrankungen oder anderen mit einer Veränderung des Methylierungsstatus assoziierten Krankheiten. Hierzu gehören u.a. CNS-Fehlfunktionen, Aggressionssymptome oder Verhaltensstörungen; klinische, psychologische und soziale Konsequenzen von Gehirnschädigungen; psychotische Störungen und Persönlichkeitsstörungen; Demenz und/oder assoziierte Syndrome; kardiovaskuläre Krankheit, Fehlfunktion und Schädigung; Fehlfunktion, Schädigung oder Krankheit des gastrointestinalen Traktes; Fehlfunktion, Schädigung oder Krankheit des Atmungssystems; Verletzung, Entzündung, Infektion, Immunität und/oder Rekonvaleszenz; Fehlfunktion, Schädigung oder Krankheit des Körpers als Abweichung im Entwicklungsprozess; Fehlfunktion, Schädigung oder Krankheit der Haut, der Muskeln, des Bindegewebes oder der Knochen; endokrine und metabolische Fehlfunktion, Schädigung oder Krankheit; Kopfschmerzen oder sexuelle Fehlfunktion. Das erfindungsgemäße Verfahren eignet sich außerdem zur Vorhersage von unerwünschten Arzneimittelwirkungen und zur Unterscheidung von Zelltypen oder Geweben oder zur Untersuchung der Zelldiffernzierung.

Erfindungsgemäß ist auch ein Kit, der aus zwei Primern, einer Polymerase sowie jeweils einer spezifischen Sonde für den methylierten und für den unmethylierten Zustand besteht sowie optional weitere für eine PCR erforderliche Reagenzien und/oder eine Bisulfitreagenz enthält.

Dem Fachmann ist bekannt, dass alle der oben genannten Ausführungsformen des erfindungsgemäßen Verfahrens nicht nur für die Methylierungsanalyse, sondern auch für die Quantifizierung von Sequenzunterschieden in RNA oder in DNA verwendet werden können. Hierfür wird der erste Schritt der beschriebenen Verfahren - die chemische oder enzymatische Umwandlung - nicht durchgeführt. So ist es möglich, mittels des erfindungsgemäßen Verfahrens eine allelspezifische Genexpression zu untersuchen oder Single Nucleotide Polymorphismen aus gepoolten Proben zu untersuchen (vgl.zur allelspezifischen Genexpression: Lo et al.: Allelic variation in gene expression is common in the human genome. Genome Res. 2003 Aug; 13 (8) : 1855-62) . Alle oben beschriebenen bevorzugten Ausführungsformen lassen sich entsprechend auf diese beiden Anwendungen außerhalb der Methylierungsanalyse übertragen und sind daher auch Teil dieser Erfindung. Das gleiche gilt für die aufgezeigten Verwendungen und den Kit. Dabei ist dem Fachmann bekannt, wie er die oben beschriebenen Verfahren modifizieren muß. So kann bei der Untersuchung der Genexpression eine Einführung einer reversen Transkription erforderlich sein.

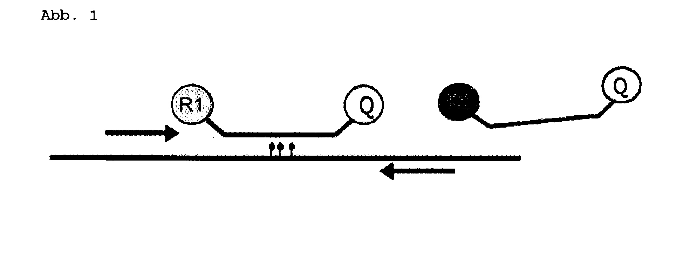

Abbildung 1: Prinzip des QM-Assays:
Für die Amplifikation werden Primer verwendet, die bisulfitspezifisch sind, aber keine CpG-Positionen (dargestellt als schwarze Kreise) beinhalten; die Sonden sind spezifisch für den methylierten bzw. den nicht-methylierten Zustand der abgedeckten CpG-Positionen; verwendet man beide Sonden in der gleichen Reaktion, so werden sie mit unterschiedlichen Fluoreszenzfarbstoffen markiert (R1, R2; Q = Quencher)

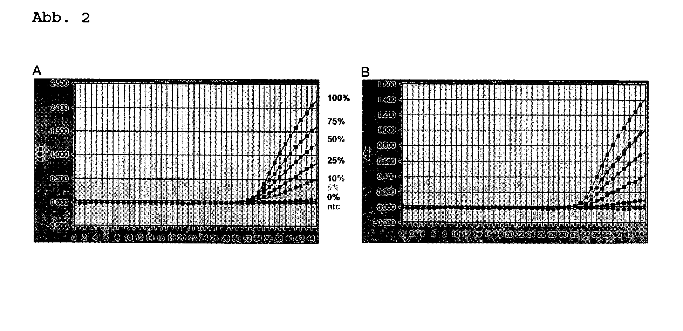

Abbildung 2: Detektion der Amplifikationsprodukte von TFF1 in jedem Zyklus (x-Achse) mittels Fluoreszenzsignalen der hybridisierten Sonden (y-Achse: Fluoreszenzintensität); A: Amplifikationskurven von DNA-Mischungen bekannten Methylierungslevels detektiert mit der FAMmarkierten Sonde für den methylierten Zustand; B: entsprechende Detektion mit der VIC-markierten Sonde für den nicht-methylierten Zustand

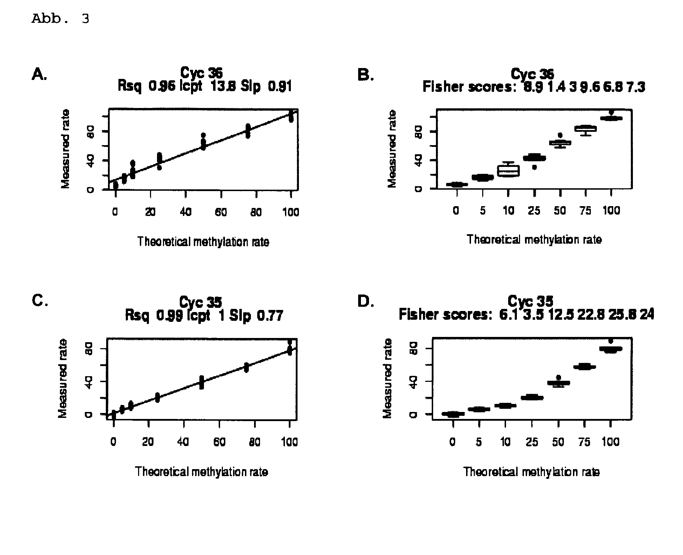

Abbildung 3: Kalibrierungskurven basierend auf Fluoreszenzintensitäten im optimalen Zyklus (Maximum der ersten Ableitung der Amplifikationskurve) und entsprechende Kurvenparameter; A, B: Zyklus 36 der Amplifkation von TFF1, 1ng Start-DNA; A: Steigung, $R^2$,

y-Achsenabschnitt; B: whisker plots von Fisher scores; C, D: Zyklus 35 der Amplifkation von S100A2, 1ng Start-DNA; C: Steigung, $R^2$, y-Achsenabschnitt D: whisker plots von Fisher scores

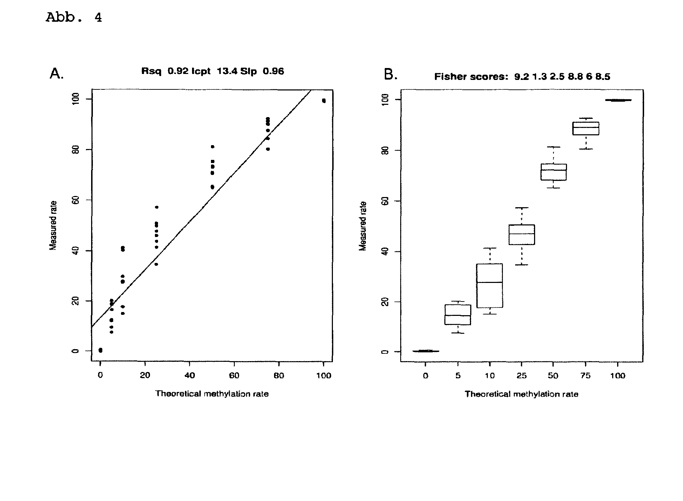

Abbildung 4: Kalibrierungskurven basierend auf Ct-Werten und entsprechende Kurvenparameter, Amplifikation von TFF1 auf 1ng DNA; A: Steigung, $R^2$, y-Achsenabschnitt; B: whisker plots von Fisher scores

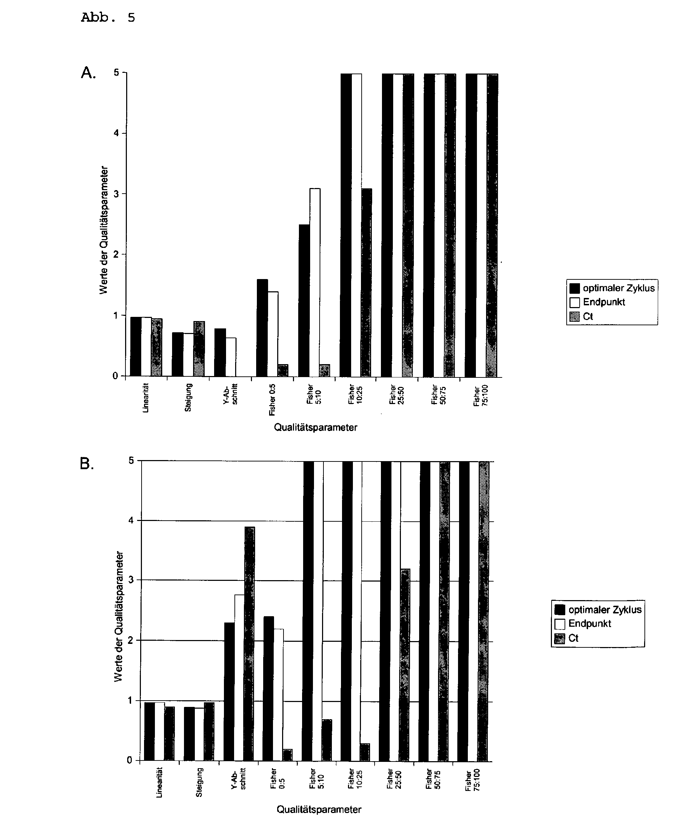

Abbildung 5: Vergleich der Kurvenparameter (Steigung, $R^2$, y-Achsenabschnitt, Fisher scores für Differenzierung benachbarter Methylierungsniveaux) der Kalibrierungskurven, die bei verschiedenen Wegen der Auswertung (basierend auf Fluoreszenzintensitäten im optimalen Zyklus oder im Endpunkt oder basierend auf Cts) von Amplifikationskurven erhalten werden; A: Amplifikation von S100A2 auf 10 ng Start-DNA; B: Amplifikation von TFF1 auf 10 ng Start-DNA.

Beispiel 1

Es soll gezeigt werden, dass mit dem erfindungsgemäßen Verfahren eine zuverlässige Quantifizierung der DNA-Methylierung möglich ist. Hierzu soll der Methylierungsgrad der beiden Gene S100A2 und TFF1 analysiert werden. Es wurden Kalibrierungskurven mit mehreren DNA-Gemischen unterschiedlicher Methylierungsgrade aufgenommen. Als Standard wurde eine Reihe von DNA-Gemischen bekannter Methylierungsgrade verwendet ( 0, 5, 10, 25, 50, 75 und 100% methylierte DNA). Zur Herstellung dieses "Goldstandards" wurde vollständig methylierte und vollständig unmethylierte DNA in unterschiedlichen Verhältnissen miteinander gemischt. Die vollständig unmethylierte DNA wurde über Molecular Staging bezogen. Dort wurde sie über eine Muliple Displacement Amplifikation humaner genomischer DNA aus Vollblut hergestellt. Die vollständig methylierte DNA wurde mittels einer Sss1-Behandlung der vollständig unmethylierten DNA entsprechend der Herstellerangaben hergestellt. Die DNA wurde anschließend bisulfitier-konvertiert (vgl.: Deutsche Patentanmeldung 10347399.8). Für die Real-Time-PCR-Assays wurden Primer-Paare verwendet, die spezifisch für die Bisulfitumwandlung waren. Die Primer waren aber methylierungsunspezifisch, d.h. sie umfassten keine CpG-Positionen. Es wurden zwei bisulfitspezifische MGB-Taqman-Sonden (Applied Biosystems) eingesetzt. Diese Sonden umfaßten 2 CpG-Positionen. Eine Sonde war spezifisch für den methylierten Zustand und war mit FAM markiert. Die zweite Sonde war spezifisch für den unmethylierten Zustand und trug eine VIC-Markierung (siehe Abb. 1). Folgende Primer und Sonden wurden für TFF1 verwendet: methylierungsspezifische Sonde: 6FAM-ACACCGTTCGTaaaa-MGBNFQ (Seq ID1) , nichtmethylierungsspezifische Sonde VIC-ACACCATTCATaaaaT-MGBNFQ (seq ID2), Forward Primer: AGtTGGTGATGtTGATtAGAGtt (Seq ID3), Reverse Primer CCC-TCCCAaTaTaCAAATAAaaaCTa (Seq ID4). Für S100-A2 wurden folgende Oligonukleotide eingesetzt: methylierungsspezifische Sonde: 6FAMtTCGTGTAtATAtATGCGttTG-MGBNFQ (Seq ID 5), nichtmethylation-specifische Sonde VICtTTGTGTAtATATATGTGttTGTG-MGBNFQ (Seq ID6), Forward Primer TttTGTGTGAGAGGtTGTGAGtAt (Seq ID7), Reverse Primer CCTCCTaATaTCCCCCAaCT (Seq IDd 8). Die Real-time-PCR wurde in einem ABI7700 Sequence Detection System (Applied Biosystems) in einem 20μl Reaktionsvolumen ausgeführt. Die Endkonzentrationen in den Reaktionsgemischen betrugen: 1xTaqMan Buffer A (Applied Biosystems) einschließlich ROX als ein passiver Referenzfarbstoff, 2.5mmol/l MgCl2 (Applied biosystems), 1 U AmpliTaq Gold DNA Polymerase (Applied Biosystems), 625nmol/l Primer, 200nmol/l Sonde, 200 μmol/l dNTP. Das Temperaturprofil für den TFF1-Assay wurde wie folgt durchgeführt: 10 min Aktivierung bei 94°C, gefolgt von 45 Zyklen von 15 s bei 94°C Denaturierung und 60 s bei 60°C Annealing + Elongation. Während des 60°C-Schritts wurde die Fluoreszenz gemessen (Abb. 2). Für den S100A2-Assay wurde das Annealing bei 62°C durchgeführt. Die Datenanalyse erfolgte entsprechend der Empfehlungen von Applied Biosystems. Die Methylierungsgrade wurden nach folgender Formel bestimmt: Methylierungsrate: = delta Rn CG-Sonde/ (delta Rn CG-Sonde + delta Rn TG-Sonde). Durch Auftragung der gemessenen Methylierungsraten gegen die theoretischen Methylierungsraten wurde eine Kalibrierungskurve für jeden PCR-Zyklus erstellt (Abb.3). Die Eignung der einzelnen Kurven für die Quantifizierung wurde über folgende Kurvenparameter bestimmt: Steigung, $R^2$, y-Achsenabschnitt sowie Fisher scores für die Klassifizierung jeweils benachbarter Methylierungslevel (Abb. 3). Von den gleichen Experimenten wurden Kalibrieungskurven basierend auf den Threshold-Zyklen (Ct) erstellt, wobei die Methylierungsrate mit folgender Formel errechnet wurde: Methylierungsrate = $100/ (1 + 2^{\text{delta Ct}}$ (Abb. 4). Vergleicht man die Eignung der verschiedenen Zyklen (optimaler Zyklus, in dem die Steigung der Amplifikationskurve maximal ist, gegen Endzyklus) und die Eignung der Kalibrierung basierend auf Ct-Werten, erkennt man, dass insgesamt die Kalibrierung über den optimalen Zyklus die besten Kurvenparameter ergibt (Abb. 5): Steigung nahe 1, $R^2$ nahe 1, y-Achsenabschnitt nahe 0, Fisher scores >1.

**Patentansprüche**

1. Verfahren zur Quantifizierung von methylierter DNA, **dadurch** gekennzeichet, dass

   a) die zu untersuchende DNA so umgesetzt wird, dass 5- Methylcytosin unverändert bleibt, während unmethy- liertes Cytosin in Uracil oder in eine andere Base umgewandelt wird, die sich im Basenpaarungsverhalten von Cytosin unterscheidet,
   b) die umgewandelte DNA in Gegenwart zweier Real-Time- Sonden amplifiziert wird, wobei die eine Sonde spe- zifisch für den methylierten Zustand und die andere Sonde spezifisch für den unmethylierten Zustand der DNA ist,

c) zu unterschiedlichen Zeitpunkten festgestellt wird, inwieweit eine Hybridisierung der Sonden an die Amplifikate erfolgt ist,

d) der Methylierungsgrad der untersuchten DNA bestimmt wird.

2. Verfahren nach Anspruch 1, **dadurch gekennzeichnet, dass** in Schritt b) ein exponentielles Amplifikationsverfahren verwendet wird.

3. Verfahren nach Anspruch 2, **dadurch gekennzeichnet, dass** eine PCR verwendet wird.

4. Verfahren nach mindestens einem der Ansprüche 1-3, **dadurch gekennzeichnet, dass** die Amplifikation mittels methylierungsunspezifischer Primern durchgeführt wird.

5. Verfahren nach mindestens einem der Ansprüche 1-4, **dadurch gekennzeichnet, dass** als Real-Time-Sonden Lightcycler-, Taqman-, Sunrise-, Molecular Beacon oder Eclipse-Sonden verwendet werden.

6. Verfahren nach Anspruch 4, **dadurch gekennzeichnet, dass** Als Real-Time-Sonde Taqman-Sonden in Kombination mit Minor Groove Bindern eingesetzt werden.

7. Verfahren nach mindestens einem der Ansprüche 1-6, **dadurch gekennzeichnet, dass** die Amplifikation in Gegenwart beider Sonden in demselben Gefäß durchgeführt wird.

8. Verfahren nach mindestens einem der Ansprüche 1-7, **dadurch gekennzeichnet, dass** der Methylierungsgrad aus dem Verhältnis der Signalintensitäten der beiden Sonden zu einem bestimmten Zeitpunkt berechnet wird.

9. Verfahren nach Anspruch 8, **dadurch gekennzeichnet, dass** der Methylierungsgrad aus dem Verhältnis der Signalintensitäten zu einem Zeitpunkt während der exponentiellen Amplifikationsphase bestimmt wird.

10. Verfahren nach Anspruch 9, **dadurch gekennzeichnet, dass** der Methylierungsgrad aus dem Verhältnis der Signalintensitäten zu einem Zeitpunkt bestimmt wird, der 5 Zyklen vor oder nach dem Zeitpunkt liegt, in dem die Amplifikation ihre maximale Steigung erreicht (Wendepunkt der Fluoreszenzintensitätskurven).

11. Verfahren nach Anspruch 10, **dadurch gekennzeichnet, dass** der Methylierungsgrad aus dem Verhältnis der Signalintensitäten zu einem Zeitpunkt bestimmt wird, der 2 Zyklen vor oder nach dem Zeitpunkt liegt, in dem die Amplifikation ihre maximale Steigung erreicht.

12. Verfahren nach Anspruch 11, **dadurch gekennzeichnet, dass** der Methylierungsgrad aus dem Verhältnis der Signalintensitäten zu einem Zeitpunkt bestimmt wird, der 1 Zyklus vor oder nach dem Zeitpunkt liegt, in dem die Amplifikation ihre maximale Steigung erreicht.

13. Verfahren nach Anspruch 12, **dadurch gekennzeichnet, dass** der Methylierungsgrad aus dem Verhältnis der Signalintensitäten zu einem Zeitpunkt bestimmt wird, in dem die Amplifikation ihre maximale Steigung erreicht (Wendepunkt der Fluoreszenzintensitätskurven).

14. Verfahren nach mindestens einem der Ansprüche 1-7, **dadurch gekennzeichnet, dass** die Berechnung des Methylierungsgrades über das Verhältnis von Schwellenwerten, bei denen eine gewisse Signalintensität überschritten wird, erfolgt.

15. Verfahren nach Anspruch 14, **dadurch gekennzeichnet, dass** die Berechnung über das Verhältnis von Ct Werten erfolgt.

16. Verfahren nach Anspruch 15, **dadurch gekennzeichnet, dass** die Berechnung über folgende Formel erfolgt: Methylierungsgrad = $100/(1+2^{\Delta Ct})$

17. Verfahren nach mindestens einem der Ansprüche 1-7, **dadurch gekennzeichnet, dass** die Berechnung des Methylierungsgrades über das Verhältnis der Fläche unter den Fluoreszenzintesitätskurven (area under the curve) oder über die maximale Steigung der Kurven erfolgt.

18. Verfahren nach mindestens einem der Ansprüche 1-17, **dadurch gekennzeichnet, dass** vor Schritt a) die Assaybedingungen so optimiert werden, dass die Fluoreszenzintensitätskurven für den Zeitpunkt der exponentiellen Amplifikation über einen möglichst geringen y-Achsenabschnitt und einen möglichst hohen Fischer-Score verfügen.

19. Verfahren nach mindestens einem der Ansprüche 1-18, **dadurch gekennzeichnet, dass** vor Schritt a) die Assaybedingungen so optimiert werden, dass die Fluoreszenzintensitätskurven für den Zeitpunkt der exponentiellen Amplifikation eine Steigung und eine Regression nahe dem Wert 1 haben.

**20.** Verfahren zur absoluten Bestimmung des Gehalts an methylierter DNA, **dadurch gekennzeichnet, dass**

a) ein Verfahren nach einem der Ansprüche 1-19 durchgeführt wird,
b) das Ergebnis mit dem einer Standardkurve verglichen wird.

**21.** Verfahren nach mindestens einem der Ansprüche 1-20,
**dadurch gekennzeichnet, dass** die Quantifizierung zur Diagnose von Krebserkrankungen oder anderen mit einer Veränderung des Methylierungsstatus assoziierten Krankheiten erfolgt.

**22.** Verfahren nach mindestens einem der Ansprüche 1-20,
**dadurch gekennzeichnet, dass** die Quantifizierung zur Vorhersage von unerwünschten Arzneimittelwirkungen und zur Unterscheidung von Zelltypen oder Geweben, oder zur Untersuchung der Zelldifferenzierung erfolgt.

**23.** Ein Kit, der aus zwei Primern, einer Polymerase sowie jeweils einer spezifischen Sonde für den methylierten und für den unmethylierten Zustand besteht sowie optional weitere für eine PCR erforderliche Reagenzien und/oder eine Bisulfitreagenz enthält

**24.** Verfahren zur Quantifizierung allelspezifische Genexpression, **dadurch gekennzeichnet, dass**:

a) die zu untersuchende RNA revers transkribiert wird
b) die cDNA in Gegenwart zweier Real-Time-Sonden amplifiziert wird, wobei die eine Sonde spezifisch für den Zustand des einen Allels und die andere Sonde spezifisch für den Zustand des anderen Allels ist,
c) zu unterschiedlichen Zeitpunkten festgestellt wird, inwieweit eine Hybridisierung der Sonden an die Amplifikate erfolgt ist,
d) die allelspezifische Genexpression quantifiziert wird

**25.** Verfahren zur Untersuchung von SNPs aus gepoolten Proben, **dadurch gekennzeichnet, dass**:

a) die zu untersuchende DNA in Gegenwart zweier Real- Time-Sonden amplifiziert wird, wobei die eine Sonde spezifisch für eine Basensequenz des SNP und die an- dere Sonde spezifisch für anderen Zustand des SNP ist,
b) zu unterschiedlichen Zeitpunkten festgestellt wird, inwieweit eine Hybridisierung der Sonden an die Amplifikate erfolgt ist,
c) auf die SNP geschlossen wird.

Abb. 1

Abb. 2

Abb. 3

**A.**

Cyc 36
Rsq 0.96 Icpt 13.8 Slp 0.91

Theoretical methylation rate

**B.**

Cyc 36
Fisher scores: 8.9 1.4 3 9.6 6.8 7.3

Theoretical methylation rate

**C.**

Cyc 35
Rsq 0.99 Icpt 1 Slp 0.77

Theoretical methylation rate

**D.**

Cyc 35
Fisher scores: 6.1 3.5 12.5 22.8 25.8 24

Theoretical methylation rate

Abb. 4

A.    Rsq 0.92 Icpt 13.4 Slp 0.96

B.    Fisher scores: 9.2 1.3 2.5 8.8 6 8.5

Abb. 5

A.

B.

Europäisches Patentamt

EUROPÄISCHER RECHERCHENBERICHT

Nummer der Anmeldung

EP 04 09 0133

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.7) |
|---|---|---|---|
| D,X | US 6 331 393 B1 (LAIRD PETER W ET AL) 18. Dezember 2001 (2001-12-18) <br> * Spalte 5, Zeile 16 - Spalte 6, Zeile 56 * <br> * Spalte 11, Zeile 5 - Spalte 16, Zeile 29 * <br> * Abbildungen 3,5b,7 * <br> * Beispiele 1,2,4 * <br> ----- | 1-23 | C12Q1/68 <br> G06F19/00 |
| X | EADS C A ET AL: "MethyLight: a high throughput assay to measure DNA methylation" <br> NUCLEIC ACIDS RESEARCH, OXFORD UNIVERSITY PRESS, SURREY, GB, <br> Bd. 28, Nr. 8, 2000, Seiten 1-8, <br> XP002962893 <br> ISSN: 0305-1048 <br> * Seite II, linke Spalte, Absatz 6 - rechte Spalte, Absatz 2 * <br> * Abbildungen 1,3,5 * <br> * Seite VIII, linke Spalte, Absatz 2 * <br> ----- | 1-23 | |
| X | RAND KEITH ET AL: "Conversion-specific detection of DNA methylation using real-time polymerase chain reaction (ConLight-MSP) to avoid false positives" <br> METHODS (ORLANDO), <br> Bd. 27, Nr. 2, Juni 2002 (2002-06), Seiten 114-120, XP002296074 <br> ISSN: 1046-2023 <br> * Seite 116, linke Spalte, Absatz 2 - rechte Spalte, Absatz 1 * <br> * Seite 118, linke Spalte, Absatz 3 - Seite 119, linke Spalte, Absatz 1 * <br> ----- <br> -/-- | 1-23 | RECHERCHIERTE SACHGEBIETE (Int.Cl.7) <br><br> C12Q |

Der vorliegende Recherchenbericht wurde für alle Patentansprüche erstellt

| Recherchenort | Abschlußdatum der Recherche | Prüfer |
|---|---|---|
| München | 15. September 2004 | Ulbrecht, M |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
..................................................................
& : Mitglied der gleichen Patentfamilie,übereinstimmendes Dokument

EPO FORM 1503 03.82 (P04C03)

EP 1 655 377 A1

**Europäisches Patentamt**

**EUROPÄISCHER RECHERCHENBERICHT**

Nummer der Anmeldung

EP 04 09 0133

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.7) |
|---|---|---|---|
| X | WO 03/081532 A (SEEING MACHINES PTY LTD ; THOMSEN COLIN (AU); LOY GARETH (AU)) 2. Oktober 2003 (2003-10-02) * Seite 11, Zeile 27 - Seite 15, Zeile 121 * * Beispiele 1-3 * | 1-23 | |
| A | OLIVER D H ET AL: "Use of single nucleotide polymorphisms (SNP) and real-time polymerase chain reaction for bone marrow engraftment analysis." THE JOURNAL OF MOLECULAR DIAGNOSTICS : JMD. NOV 2000, Bd. 2, Nr. 4, November 2000 (2000-11), Seiten 202-208, XP002296075 ISSN: 1525-1578 * Seite 203, linke Spalte, Absatz 2 - rechte Spalte, Absatz 1 * | 1-23 | |
| A | LIVAK K J: "ALLELIC DISCRIMINATION USING FLUOROGENIC PROBES AND THE 5' NUCLEASEASSAY" GENETIC ANALYSIS: BIOMOLECULAR ENGINEERING, ELSEVIER SCIENCE PUBLISHING, US, Bd. 14, 1999, Seiten 143-149, XP002944060 ISSN: 1050-3862 * das ganze Dokument * | 1-23 | RECHERCHIERTE SACHGEBIETE (Int.Cl.7) |

Der vorliegende Recherchenbericht wurde für alle Patentansprüche erstellt

| Recherchenort | Abschlußdatum der Recherche | Prüfer |
|---|---|---|
| München | 15. September 2004 | Ulbrecht, M |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie,übereinstimmendes Dokument

EPO FORM 1503 03.82 (P04C03)

**Europäisches**
**Patentamt**

Nummer der Anmeldung

EP 04 09 0133

---

## GEBÜHRENPFLICHTIGE PATENTANSPRÜCHE

Die vorliegende europäische Patentanmeldung enthielt bei ihrer Einreichung mehr als zehn Patentansprüche.

☐ Nur ein Teil der Anspruchsgebühren wurde innerhalb der vorgeschriebenen Frist entrichtet. Der vorliegende europäische Recherchenbericht wurde für die ersten zehn sowie für jene Patentansprüche erstellt, für die Anspruchsgebühren entrichtet wurden, nämlich Patentansprüche:

☐ Keine der Anspruchsgebühren wurde innerhalb der vorgeschriebenen Frist entrichtet. Der vorliegende europäische Recherchenbericht wurde für die ersten zehn Patentansprüche erstellt.

---

## MANGELNDE EINHEITLICHKEIT DER ERFINDUNG

Nach Auffassung der Recherchenabteilung entspricht die vorliegende europäische Patentanmeldung nicht den Anforderungen an die Einheitlichkeit der Erfindung und enthält mehrere Erfindungen oder Gruppen von Erfindungen, nämlich:

Siehe Ergänzungsblatt B

☐ Alle weiteren Recherchengebühren wurden innerhalb der gesetzten Frist entrichtet. Der vorliegende europäische Recherchenbericht wurde für alle Patentansprüche erstellt.

☐ Da für alle recherchierbaren Ansprüche die Recherche ohne einen Arbeitsaufwand durchgeführt werden konnte, der eine zusätzliche Recherchengebühr gerechtfertigt hätte, hat die Recherchenabteilung nicht zur Zahlung einer solchen Gebühr aufgefordert.

☐ Nur ein Teil der weiteren Recherchengebühren wurde innerhalb der gesetzten Frist entrichtet. Der vorliegende europäische Recherchenbericht wurde für die Teile der Anmeldung erstellt, die sich auf Erfindungen beziehen, für die Recherchengebühren entrichtet worden sind, nämlich Patentansprüche:

☒ Keine der weiteren Recherchengebühren wurde innerhalb der gesetzten Frist entrichtet. Der vorliegende europäische Recherchenbericht wurde für die Teile der Anmeldung erstellt, die sich auf die zuerst in den Patentansprüchen erwähnte Erfindung beziehen, nämlich Patentansprüche:

1-23

Europäisches
Patentamt

**MANGELNDE EINHEITLICHKEIT
DER ERFINDUNG
ERGÄNZUNGSBLATT B**

Nummer der Anmeldung

EP 04 09 0133

Nach Auffassung der Recherchenabteilung entspricht die vorliegende europäische Patentanmeldung nicht den Anforderungen an die Einheitlichkeit der Erfindung und enthält mehrere Erfindungen oder Gruppen von Erfindungen, nämlich:

1. Ansprüche: 1-23

   Verfahren und Kit zur Quantifizierung methylierter DNA

   ---

2. Anspruch: 24

   Verfahren zur Quantifizierung allelspezifischer Genexpression

   ---

3. Anspruch: 25

   Verfahren zur Untersuchung von SNPs aus gepoolten Proben.

   ---

**ANHANG ZUM EUROPÄISCHEN RECHERCHENBERICHT
ÜBER DIE EUROPÄISCHE PATENTANMELDUNG NR.**                EP 04 09 0133

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

15-09-2004

| Im Recherchenbericht angeführtes Patentdokument | | Datum der Veröffentlichung | Mitglied(er) der Patentfamilie | | Datum der Veröffentlichung |
|---|---|---|---|---|---|
| US 6331393 | B1 | 18-12-2001 | AU | 775798 B2 | 19-08-2004 |
| | | | AU | 4712200 A | 05-12-2000 |
| | | | CA | 2372665 A1 | 23-11-2000 |
| | | | EP | 1185695 A1 | 13-03-2002 |
| | | | JP | 2002543852 T | 24-12-2002 |
| | | | WO | 0070090 A1 | 23-11-2000 |
| | | | US | 2002086324 A1 | 04-07-2002 |
| WO 03081532 | A | 02-10-2003 | WO | 03081532 A1 | 02-10-2003 |
| | | | AU | 2003212095 A1 | 08-10-2003 |

Für nähere Einzelheiten zu diesem Anhang : siehe Amtsblatt des Europäischen Patentamts, Nr.12/82